# EUROPEAN PATENT APPLICATION

(11) **EP 4 552 742 A1**
(43) Date of publication of application: **14.05.2025**
(21) Application number: 24211795.0
(22) Date of filing: 08.11.2024
(51) Int. Cl.: B01L 3/00, A61B 10/00, G01N 1/02

(54) **SPECIMEN COLLECTOR LID HAVING DETACHABLE HANDLE AND METHOD**

(30) Priority: 10.11.2023 US 202363548109 P
(71) Applicant: Nguyen, Phuong, San Diego, CA 92108 (US)
(72) Inventor: Nguyen, Phuong, San Diego, CA 92108 (US)
(74) Representative: Viering, Jentschura & Partner mbB Patent- und Rechtsanwälte

(57) **Abstract**

A specimen collection, storage, transport, and testing device can include a vessel carrying a liquid reagent. A lid sealing the vessel can include a solid specimen collection stick projecting from an inside surface of the lid. Further, the lid can include a port on its outside surface. The port can be releasably engaged by a hand-manipulable handle facilitating specimen collection and attachment of the lid to the vessel. The port can further include an openably sealed aperture for accommodating the insertion of a test strip-carrying cartridge. The reusability of the handle and its reduced material structure helps avoid waste.

## Description

### Field of the Invention

The invention relates to immunoassay devices for conducting chromatographic testing of liquid and liquid immersible specimens, and more particularly to devices for collection, storage, and preliminary screening of materials such as pathological, forensic, and environmental specimens.

### Background

Liquid specimen testing containers are commonly used to collect and test liquid specimens for the presence or absence of specific "indicators" which show the presence of certain chemicals, hormones, antibodies or antigens associated with various physiological conditions and are commonly used for drug abuse screening. Such containers can also be used to store and transport portions of the specimen to a lab for subsequent, more rigorous, confirmatory testing. Such containers can also be adapted to test semi-solid material specimens such as bodily excretions, gels, and powders by mixing the specimen with one or more liquid reagents within the container. For example, devices such as shown in Nguyen, US Pat. No. 7981054 provide for testing fecal material specimens among other possible specimens.

As disclosed in Vallejo, et al., US Pat. No. 7507373, the type of preliminary screening test being conducted can be easily changed by replacing the strip-containing cartridge with one carrying a different panel of strips designed to detect a different set of indicators. Such flexibility can be important so that the same device can be used for many different types of tests, thereby reducing manufacturing and distribution costs.

Another potential problem involves the timing of the initiation of the test. Often, the results of the preliminary screening test may be valid for a narrow span of time. Thus, it can be useful to prevent the donor from initiating the preliminary screening test and instead allow the test to be conducted by a qualified technician. This also can help prevent donor tampering.

As disclosed in Cipkowsky, US Pat. No. 5976895, a preliminary screening test can be initiated at some time after a specimen has been collected by inserting a test strip-carrying cartridge through a slot on the specimen container lid. Furthermore, a decision of the type of test to be conducted can be decided at any time prior to initiation of the test. In this way potential tampering by the test subject can be reduced. Also, the health risk associated with exposure of the specimen to the testing technician can be reduced or avoided altogether.

Because such devices can become contaminated by the specimens the above devices are often made disposable after a single use, creating large amounts of waste, leading to increased environmental stress. Thus, there is an incentive to reduce the waste associated with such devices. As such, there is a trend to reduce the size of preliminary screening testing devices. Reduced size however can adversely impact the ease of use during handling.

Increasingly, preliminary screening tests are being performed and evaluated by relatively unskilled technicians or even the general public. Therefore, the device needs to be relatively simple to operate to ensure adequate exposure of the preliminary test strips and to provide more consistent results.

Therefore there is a need for a specimen test device which addresses some or all of the above identified inadequacies.

### Summary

The principal and secondary objects of the invention are to help provide an improved specimen collection, preliminary screening, storage, and transport device. These and other objects are achieved by a small vessel having a multipurpose lid including a detachable handle and aperture for the insertion of a testing cartridge.

In some embodiments there is provided an assay device for testing a specimen, said device comprises: a vessel which comprises: a top opening leading to an inner chamber; a lid sealing said top opening; a handle removably secured to said lid; wherein said lid comprises: an outer surface and an inner surface; a collector stick having an upper end secured to said inner surface; a port formed into said outer surface; wherein said port comprises: a receptacle releasably securing to a fastening engagement on said handle; an aperture sealed by a frangible barrier; wherein said aperture is shaped and dimensioned to allow insertion of a test-strip carrying cartridge therethrough.

In some embodiments said handle comprises: a substantially cylindrical body having a central axis and a maximum radius; a plurality of vanes extending substantially radially with respect to said axis; and, wherein said plurality of vanes are angularly spaced apart from one another.

In some embodiments each of said vanes has a radial dimension that is greater than one half of a maximum radius of said handle.

In some embodiments said device further comprises: a first one of said plurality of vanes; a second one of said plurality of vanes being angularly adjacent to said first one of said plurality of vanes; and, wherein said first and second ones of said plurality of vanes are separated by an angle.

In some embodiments said angle has a range of between 10 degrees and 90 degrees.

In some embodiments said plurality of vanes are uniformly angularly spaced apart.

In some embodiments a first one of said plurality of vanes has an angularly uniform width along a majority of its radial dimension; and has a rounded distal terminus whereby the vane has a substantially teardrop shape.

In some embodiments said device further comprises: said handle comprising a threaded section proximal to said proximal end; said port comprising a threaded receptacle; and, wherein said threaded section threadingly engages said threaded receptacle.

In some embodiments said device further comprises: a cartridge comprising at least one chromatographic testing strip; said port comprising an aperture formed into a bottom of said receptacle; said aperture being shaped and dimensioned to be penetrated by a portion of said cartridge.

In some embodiments said aperture is sealed by a frangible barrier.

In some embodiments said cartridge comprises: an elongated prong having an elongation axis; wherein said elongated prong has a cross-section taken substantially perpendicularly to said elongation axis; wherein said cross-section is shaped and dimensioned to be commensurate with said aperture; and, wherein said prong comprises a sharpened tip oriented to puncture said frangible barrier during insertion of said prong through said aperture.

In some embodiments said cartridge further comprises: a side panel carrying an informational placard; and, a top grasping panel.

In some embodiments said receptacle and said aperture are substantially coaxial.

In some embodiments said device further comprises: said lid comprising a substantially cylindrical threaded wall portion having a central axis; wherein said port is intersected by said central axis;wherein said collector stick is offset from said central axis.

In some embodiments said collector stick comprises: a lower end opposite said upper end; and, one or more radial disuniformities near said lower end whereby a semi-solid material can be collected in said one or more radial disuniformities.

In some embodiments said one or more radial disuniformities comprise a collector spoon near said tip wherein said spoon is dimensioned to collected a given volume of said semi-solid material.

In some embodiments it is provided that in an immunoassay flow testing device having a fluid specimen accepting vessel having a top opening sealable by a lid, and at least one chromatographic testing strip cartridge for exposure to an internal compartment of the vessel, an improvement which comprises: a collector stick secured to said lid; and, a handle removably secured to said lid.

In some embodiments there is provided an assay device for testing a first and second specimens, said device comprises: a first vessel, and a second vessel; a first lid for sealing said first vessel, and a second lid for sealing said second vessel; a handle removably securable to said first lid and said second lid; wherein each of said lids comprises: an outer surface and an inner surface; a collector stick having an upper end secured to said inner surface; a port formed into said outer surface;wherein said port comprises: a receptacle releasably securing to a fastening engagement on said handle; an aperture sealed by a frangible barrier;wherein said aperture is shaped and dimensioned to allow insertion of a test-strip carrying cartridge therethrough.

In some embodiments there is provided a method for conducting a preliminary fluid specimen test from a fluid specimen, said method comprises: selecting a device including: a vessel containing a liquid reagent, a lid having a collector stick, and a handle releasably secured to a receptacle in said lid; grasping said lid by the handle and collecting a semi-solid specimen; contacting said specimen with said reagent; sealing said vessel with said lid; removing said handle; inserting a cartridge carrying a testing strip through an aperture in said lid; contacting said reagent with said cartridge, thereby allowing a first amount of said fluid specimen to flow onto said testing strip; observing a result on said testing strip through a translucent sidewall of said vessel.

In some embodiments said method further comprises: said selecting comprising: choosing said lid to have said aperture located in a bottom of said receptacle; said inserting comprising: orienting a prong on said cartridge with said receptacle; pushing said prong into said receptacle and through a frangible barrier sealing said aperture.

In some embodiments said collecting, said sealing, and said removing occurs through manipulating said handle.

In some embodiments said method further comprises: choosing said device to be a first device, said vessel to be a first vessel, and said lid to be a first lid, and said collector stick to be a first collector stick; choosing a second device including a second vessel containing an amount of liquid reagent, and a second lid having a second collector stick; and, attaching said handle to said second lid after said removing.

The original text of the original claims is incorporated herein by reference as describing features in some embodiments.

### Brief Description of the Drawings

**Fig. 1** is a diagrammatic, perspective view of an assembled testing device according to an exemplary embodiment of the invention.
**Fig. 2** is a diagrammatic, exploded, perspective view of the testing device of Fig. 1 without the cartridge.
**Fig. 3** is a diagrammatic bottom view of the lid.
**Fig. 4** is a diagrammatic cross-sectional side view of the lid and engaged handle.
**Fig. 5** is a diagrammatic cross-sectional side view of the lid secured to the vessel, and engaged cartridge during a preliminary screening test.
**Fig. 6** is a diagrammatic, perspective view of the detached handle.
**Fig. 7** is a diagrammatic cross-sectional bottom view of the handle of Fig. 6 taken along line 7-7.
**Fig. 8** is a diagrammatic cross-sectional bottom view of a handle having four vanes according to an alternate embodiment of the invention.
**Fig. 9** is a diagrammatic cross-sectional bottom view of a handle having thirty-six vanes according to an alternate embodiment of the invention.
**Fig. 10** is a flow diagram of method steps according to an exemplary embodiment of the invention.

### Description of the Exemplary Embodiments

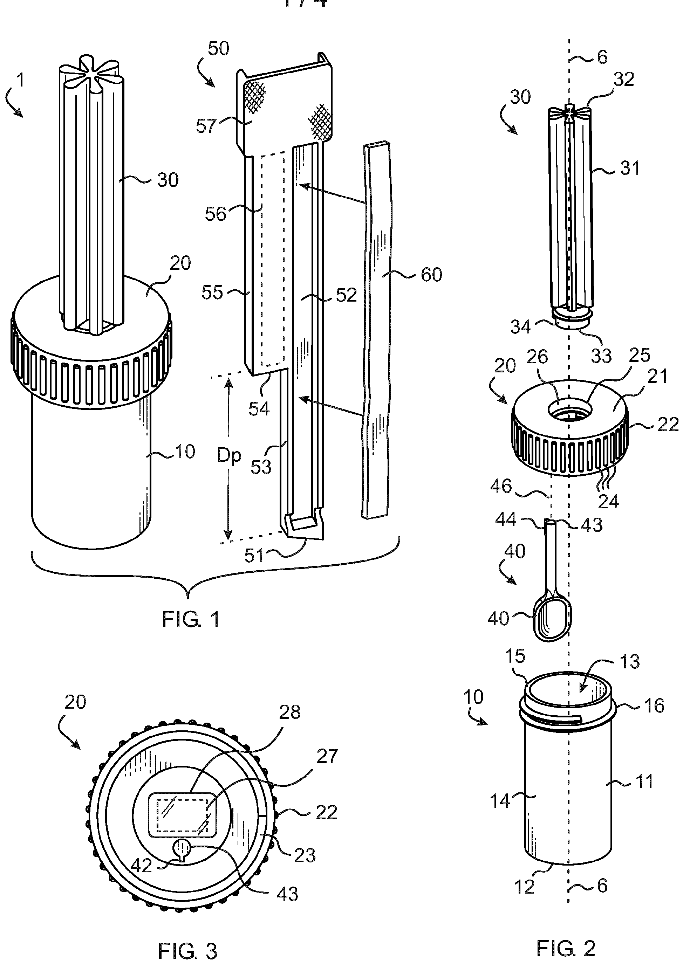

In this specification, the references to top, bottom, upward, downward, upper, lower, vertical, horizontal, sideways, lateral, back, front, etc. can be used to provide a clear frame of reference for the various structures with respect to other structures while the testing container is in its upright orientation as shown in Fig. 1, and not treated as absolutes when the frame of reference is changed, such as when the container is laying on its side.

In this specification, features described in connection with one embodiment may be applicable to the other embodiments depending on their functional requirements and restrictions. Those skilled in the art will readily appreciate that applicability.

The term "substantially" can be used in this specification because manufacturing imprecision and inaccuracies can lead to non-symmetricity and other inexactitudes in the shape, dimensioning and orientation of various structures. Further, use of "substantially" in connection with certain geometrical shapes, such as "cylindrical", "conical", and "circular", and orientations, such as "parallel" and "perpendicular", can be given as a guide to generally describe the function of various structures, and to allow for slight departures from exact mathematical geometrical shapes and orientations, while providing adequately similar function. Those skilled in the art will readily appreciate the degree to which a departure can be made from the mathematically exact geometrical references.

In this specification, the word "axial" is meant to refer to directions, movement, or forces acting substantially parallel with or along a respective axis, and not to refer to rotational nor radial nor angular directions, movement or forces, nor torsional forces. The terms "angular"and "radial" are in reference to the respective axis.

In this specification the units "millimeter" or "millimeters" can be abbreviated "mm", "centimeter" or "centimeters" can be abbreviated "cm", and "milligram" or "milligrams" can be abbreviated "mg". Units of temperature such as "degrees centigrade" can be abbreviated "°C".

Referring now to the drawing there is shown in Figs. 1-7 the components of a specimen collection, testing, transport, and storage device 1 for preliminarily screening a specimen such as an amount of fecal material for the presence of disease or abused drugs. In this embodiment, the device is specifically designed for use in connection with fecal specimens but could be readily adapted to collect urine, saliva, other readily available bodily fluids, or various other materials such as food for contamination testing, or dust for the presence of illegal substances, for example.

As shown primarily in Figs. 1-5 the device **1** can include a substantially cylindrical liquid container vessel **10** carrying an amount of a liquid reagent **17**. A lid **20** including a specimen collection stick **40** attached to its underside can be used to collect a specimen without touching it. A detachable handle **30** can extend from the top of the lid. The handle can help the user manipulate the stick to collect the specimen, and can help the user to screw the lid onto the vessel, exposing the specimen to the reagent and sealing the top of the vessel. The handle can then be detached from a port **25** on the lid exposing an aperture **27** through the lid that can be sealed by a frangible barrier **28**. The barrier can be punctured by the sharpened prong **51** of a cartridge **50** carrying a test strip **60** in order to conduct a preliminary screening test of the specimen. The handle can be reused with compatible devices, thus reducing waste.

As shown primarily in Fig. 2, the vessel **10** can have a closed substantially circular bottom **12** and an opposite substantially circular top opening **13** surrounded by a substantially circular upper lip **15** and joined to the bottom along a central axis **6** by a substantially cylindrical sidewall **11** thus enclosing a substantially cylindrical internal compartment. An external threaded region **16** can be formed on the outer surface of the sidewall near the top opening. The vessel can be made of a translucent material so that the sidewall forms a window **14** through which the internal compartment can be viewed while the vessel remains sealed. Alternately, the vessel can be made from more environmentally friendly materials such as paper, wax coated paper, cardboard, wax-coated cardboard, or other materials, where the threads may or may not be formed from plastic, metal or other materials.

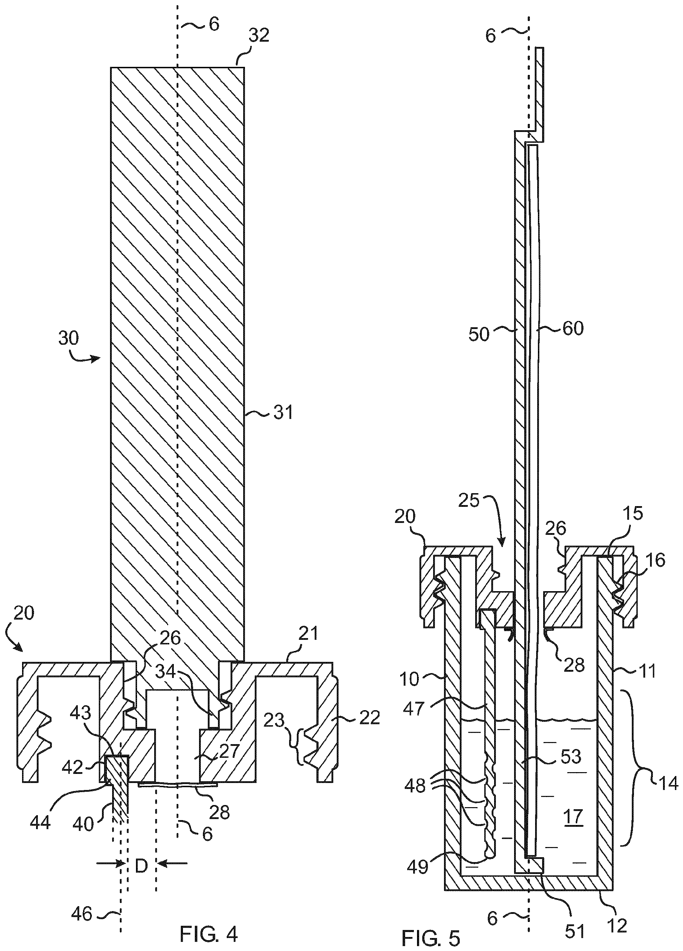

As shown primarily in Figs. 2 and 4, the lid **20** can seal the top opening **13** of the vessel 10. The lid can have a substantially circular top surface **21**. A substantially cylindrical skirt **22** can extend downwardly from the periphery of the top surface. The radially inward surface of the skirt can have an internally threaded region **23** shaped to cooperate with the external threaded region **16** on the vessel **10**, whereby rotating the lid with respect to the vessel can seal the vessel. Rotation of the lid can be facilitated by friction increasing ridges **24** and by use of a detachable handle **30** that can fasten to a port **25** accessible from the top surface of the lid as will be further described below.

The handle **30** can be formed by an axially oblong body **31** having a top end **32** and an opposite bottom end **33** having a substantially cylindrical engagement **34** that is shaped and dimensioned to releasably secure to the port **25** on the lid **20**. In this embodiment, the port can include a substantially cylindrical, internally threaded receptacle **26** to be intimately engaged by the externally threaded engagement in order to releasably fasten the handle to the lid. In order to conveniently apply torque to the lid, the handle can be substantially coaxial with the lid, thus having the engagement and receptacle in substantial axial alignment. It is noted that the threading in the receptacle and on the engagement of the handle can be left-handed threads, whereas the threading on the vessel and skirt of the lid can be right-handed. In this way, once the lid is sufficiently tightly screwed upon the vessel, additional torque on the handle will loosen the handle from the lid while allowing the lid to remain tightly screwed upon the vessel.

Alternately, other means common in the art may be used for releasably fastening the handle to the lid. These can include snap fittings for example that can include angularly keyed surfaces which can transmit torque from the handle to the lid while attached, but can attach and detach from the lid in absence of the application of torque.

The specimen collection stick **40** can attach to a well **41** formed into the underside of the lid **20**. The well can be angularly keyed with respect to the axis **46** of the stick by including a single radial notch **42**. The upper end **43** of the stick can have a corresponding radial extension **44** which causes the stick to engage the well in a unique angular orientation. The radial position of the well with respect to the axis **6** of the lid can be selected to avoid interference between the stick and the strip-carrying cartridge when it is inserted into the vessel. Such avoidance can be obtained by locating the well a radial distance **D** from the radial position of closest edge of the aperture **27**. In this way the stick and cartridge can be prevented from coming into contact with one another. It shall be understood that for those devices where the aperture is not located on the centerline, that the stick can be located there, or elsewhere. Further, for those devices where no cartridge is to be inserted, the stick can be located to extend downwardly from essentially any part of the lid that does not interfere with the vessel.

As shown primarily in Figs. 3-4, the port **25** of the lid **20** can be further adapted to include an aperture **27** through the bottom of the receptacle **26**. The aperture can be sealed by a frangible barrier **28** made from a material such as plastic-backed foil adhesively sealed over the bottom of the aperture. The intact barrier can prevent passage of liquid reagent and suspended specimen through the aperture.

As shown primarily in Figs. 1 and 5, the test strip-carrying cartridge **50** can mount at least one chromatographic test strip **60** within an oblong vertical bed **52**. A lower portion of the bed can extend into an elongated prong portion **53** having a sharpened lower tip **51** oriented to puncture the frangible barrier **28** sealing the aperture **27**. The prong portion can be elongated along a substantially vertical axis which can be coaxial with the axis **6** of the lid when the cartridge is fully inserted. A stop surface **54** located an axial distance **Dp** from the tip of the prong can stop the downward axial movement of the cartridge once the prong is fully inserted into vessel. The distance can be selected to ensure that the tip **51** of the prong does not contact the bottom **12** of the vessel **10** when the cartridge is fully engaged. The prong **53** can have a cross-section taken substantially perpendicularly to the axis **6** which is commensurate with the aperture **27** thereby allowing snug penetration therethrough. The cartridge can also include a side panel **55** for carrying an informational placard **56** identifying the test being run, and a top grasping panel **57** to provide a convenient grasping point for insertion of the prong through the aperture by the user.

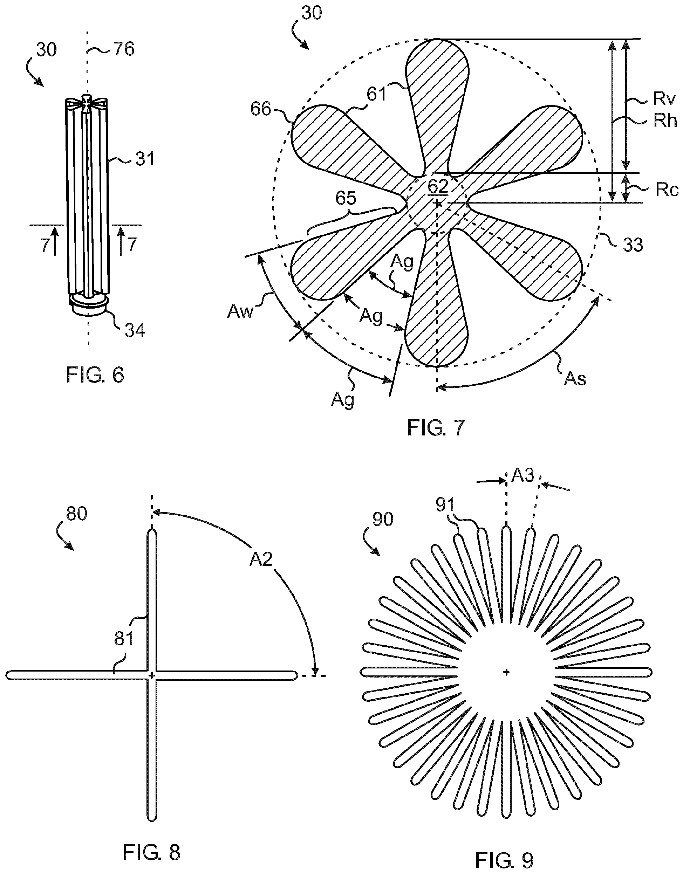

As shown in Figs. 6-7 the handle **30** can have a structure which allows detachability and reusability along with a reduction in material in order to avoid waste. The oblong body **31** of the handle **30** can extend along a central axis **76** that can be coaxial with the lid to which it secures. The body can have a roughly substantially cylindrical shape formed by a plurality of angularly spaced apart longitudinal vanes **61** extending radially from a central core **62**. Thus the radially distant tips of the vanes form the radial extent of the handle and can lie substantially within a cylinder **33**. In this embodiment there are six vanes spaced angularly evenly apart by a common angle **As** of substantially 60 degrees.

In order to minimize the amount of material in the handle **30**, each of the vanes **61** can have a radial dimension **Rv** that is at least 50% of the overall radius **Rh** of the handle. In other words, each vane can have a radial dimension **Rv** extending from the core **62** to a radial tip **64**. That radial dimension can be greater than or equal to the radius **Rc** of the core. In this embodiment the radial dimension **Rv** of each vane is approximately 4.5 times the radius of the core. In order to maintain ruggedness while minimizing material the radial dimension of each vane can range between about 1 and 10 times the radius of the core, and for most applications between about 2 and 6 times the radius of the core.

The shape of the vanes can be selected to be substantially teardrop shaped where the angular width **Aw** of each vane extending radially along a medial section **65** is substantially constant. In other words, the medial sides of each vane form a plane that, if extended radially inwardly, would intersect the central axis **76**. In this way the medial side surfaces of adjacent vanes can be separated by a constant angle **Ag**. Thus **Aw** + **Ag** = **As** which in this embodiment measures substantially 60 degrees. The distal terminus or radially distant tips **66** of each vane can be rounded to facilitate manufacturing and ease of use.

Fig. 8 shows that a handle **80** can have as few as four vanes **81** evenly angularly spaced apart by an angle **A2** of substantially 90 degrees.

Fig. 9 shows that a handle **90** can have as many as thirty-six vanes **91** evenly angularly spaced apart by an angle **A3** of substantially 10 degrees.

An advantage of using a handle having angularly spaced apart vanes is that a larger amount of friction can be formed with the user's fingers and thumb and thus greater torque can be easily applied to the lid without risk of spilling or mishandling the device. In this way, the vanes can use a minimum of material while providing a relatively large contact surface for the user's fingers. In addition there may be no undercut region which can make injection molding difficult.

The dimension of the various structures can be readily adjusted according to various parameters such as manufacturing cost, reduced bulk, and flexibility for the number of test configurations available. For example, a vessel having a volume of between about 1 and 3 milliliter, the height of the handle can be between about 150 and 500 millimeter, and for many typical fecal specimen testing applications, between about 250 and 350 millimeter.

As shown in Fig. 5, it shall be understood that for testing semi-solid materials, the collection stick **47** can have one or more radial disuniformities such as flutes **48** which can be formed into the stick near the tip **49** to allow for the capture of semi-solid material therein.

It shall be understood that the above described arrangement of elements allows for the manufacture of the components using simple injection molding techniques from common materials such as PTFE plastics and a minimum amount of assembly which can be readily automated. Indeed, the lid, handle, vessel, and cartridge can each be made from a single, unitary injection molded or 3D printed pieces of material.

Typically, after the testing is complete, the vessel, lid, collection stick and cartridge which have come into contact with the specimen can be discarded. However, because the handle does not contact the potentially infectious specimen or reagent chemicals, and is not damaged during the process of attaching and detaching it from the lid, the handle can be reused on a number of other test devices. In this way the handle can be reused on a second lid and vessel.

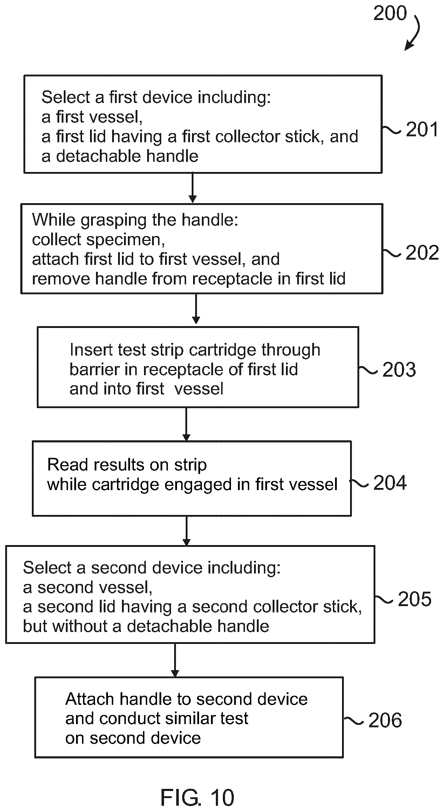

Referring now to Fig. 10 there will be described the method **200** of conducting a pair of preliminary screening tests using a pair of devices similar to that shown in Figs. 1-4. In this example of the method, both devices can be configured for collecting and testing fecal specimens. Thus, a first device is selected 201 having a first vessel, a first lid including a collector stick and a detachable handle. The first device can be delivered to the donor with an amount of reagent contained in the first vessel.

Using the first lid, the donor can collect a specimen, secure the first lid to the vessel, and remove the handle by manipulating it using the handle only **202**. Once the first lid is secured to the vessel, the semi-solid material carried on the lower end of the collector stick is immersed into the reagent and mixes with it.

At this point the cartridge carrying the testing strip can be inserted **203** through the aperture in the first lid and into the specimen inside the first vessel, initiating the preliminary screening test. After waiting a period for the strip to react, the user can view **204** the results on the cartridge.

Once the handle has been removed from the device, the handle can be used to conduct a another test with a separate device. Thus, the user can select **205** a second device similar to the first, including a second vessel, a second lid having a collector stick, but without a handle.

The user can attach **206** the handle used in the first test to the second lid and conduct a similar test using the above steps.

Alternately, the donor can then return the device containing the specimen to the lab where a technician can initiate the test. By having no access to the strip-carrying cartridge, the donor cannot accidentally or intentionally initiate the preliminary screening test. In addition, it is irrelevant whether the donor tilts or shakes the device since the specimen is completely trapped within the internal compartment of the vessel by the lid and intact barrier.

In this way the analytical process can be performed in the absence of any contacting or manipulating of the lid with respect to the vessel and specimen collector stick.

Optionally, the device can be stored and/or transported for later confirmatory testing. It is important to note that in this arrangement of elements, the axial location of the cartridge prong and radial location of the collection stick can be selected so that they do not interfere with one another.

The preliminary screening test using the above described device can easily be conducted by less skilled workers or even the general public. Thus the device can be sold commercially in drug stores and be available to a much wider market.

A plural number of different types of collection sticks can be provided as a kit. For example the stick of Fig. 2 can be provided together with the stick of Fig. 5 so that the user can decide which to use given the test to be conducted. Other types of collector sticks may be used. For example, the spoon-type collector of Fig. 2 can be removed and replaced with a sponge-type collector useful for collecting saliva.

The above structures allow for coarser tolerances in manufacturing. It shall be understood that the interface between the vessel and lid, and between the lid and cartridge can also include additional seal enhancing structures such as o-rings or various resiliently compressible structures.

While the exemplary embodiments of the invention have been described, modifications can be made and other embodiments may be devised without departing from the spirit of the invention and the scope of the appended claims.

## Claims

1. An assay device for testing a specimen, said device comprises:
a vessel which comprises:
a top opening leading to an inner chamber;
a lid sealing said top opening;
a handle removably secured to said lid;
wherein said lid comprises:
an outer surface and an inner surface;
a collector stick having an upper end secured to said inner surface;
a port formed into said outer surface;
wherein said port comprises:
a receptacle releasably securing to a fastening engagement on said handle;
an aperture sealed by a frangible barrier;
wherein said aperture is shaped and dimensioned to allow insertion of a test-strip carrying cartridge therethrough.

2. The device of Claim 1, wherein said handle comprises:
a substantially cylindrical body having a central axis and a maximum radius;
a plurality of vanes extending substantially radially with respect to said axis; and,
wherein said plurality of vanes are angularly spaced apart from one another.

3. The device of Claim 2, wherein each of said vanes has a radial dimension that is greater than one half of a maximum radius of said handle.

4. The device of Claim 2, which further comprises:
a first one of said plurality of vanes;
a second one of said plurality of vanes being angularly adjacent to said first one of said plurality of vanes; and,
wherein said first and second ones of said plurality of vanes are separated by an angle;
wherein said angle has a range of between 10 degrees and 90 degrees.

5. The device of Claim 2, wherein said plurality of vanes are uniformly angularly spaced apart; and, wherein a first one of said plurality of vanes has an angularly uniform width along a majority of its radial dimension; and has a rounded distal terminus whereby the vane has a substantially teardrop shape.

6. The device of Claim 1, which further comprises:
said handle comprising a threaded section proximal to said proximal end;
said port comprising a threaded receptacle; and,
wherein said threaded section threadingly engages said threaded receptacle.

7. The device of Claim 6, which further comprises:
a cartridge comprising at least one chromatographic testing strip;
said port comprising an aperture formed into a bottom of said receptacle;
said aperture being shaped and dimensioned to be penetrated by a portion of said cartridge;
wherein said cartridge comprises:
an elongated prong having an elongation axis;
wherein said elongated prong has a cross-section taken substantially perpendicularly to said elongation axis;
wherein said cross-section is shaped and dimensioned to be commensurate with said aperture; and,
wherein said prong comprises a sharpened tip oriented to puncture said frangible barrier during insertion of said prong through said aperture;
a side panel carrying an informational placard; and,
a top grasping panel.

8. The device of Claim 6, which further comprises:
a cartridge comprising at least one chromatographic testing strip;
said port comprising an aperture formed into a bottom of said receptacle;
said aperture being shaped and dimensioned to be penetrated by a portion of said cartridge; and,
wherein said receptacle and said aperture are substantially coaxial.

9. The device of Claim 1, which further comprises:
said lid comprising a substantially cylindrical threaded wall portion having a central axis;
wherein said port is intersected by said central axis;
wherein said collector stick is offset from said central axis.

10. The device of Claim 1, wherein said collector stick comprises:
a lower end opposite said upper end; and,
one or more radial disuniformities near said lower end whereby a semi-solid material can be collected in said one or more radial disuniformities.

11. The device of Claim 10, wherein said one or more radial disuniformities comprise a collector spoon near said tip wherein said spoon is dimensioned to collected a given volume of said semi-solid material.

12. An assay device for testing a first and second specimens, said device comprises:
a first vessel, and a second vessel;
a first lid for sealing said first vessel, and a second lid for sealing said second vessel;
a handle removably securable to said first lid and said second lid;
wherein each of said lids comprises:
an outer surface and an inner surface;
a collector stick having an upper end secured to said inner surface;
a port formed into said outer surface;
wherein said port comprises:
a receptacle releasably securing to a fastening engagement on said handle;
an aperture sealed by a frangible barrier;
wherein said aperture is shaped and dimensioned to allow insertion of a test-strip carrying cartridge therethrough.

13. A method for conducting a preliminary fluid specimen test from a fluid specimen, said method comprises:
selecting a device including: a vessel containing a liquid reagent, a lid having a collector stick, and a handle releasably secured to a receptacle in said lid;
grasping said lid by the handle and collecting a semi-solid specimen;
contacting said specimen with said reagent;
sealing said vessel with said lid;
removing said handle;
inserting a cartridge carrying a testing strip through an aperture in said lid;
contacting said reagent with said cartridge, thereby allowing a first amount of said fluid specimen to flow onto said testing strip;
observing a result on said testing strip through a translucent sidewall of said vessel.

14. The method of Claim 13, which further comprises:
said selecting comprising:
choosing said lid to have said aperture located in a bottom of said receptacle;
said inserting comprising:
orienting a prong on said cartridge with said receptacle;
pushing said prong into said receptacle and through a frangible barrier sealing said aperture;
wherein said collecting, said sealing, and said removing occurs through manipulating said handle.

15. The method of Claim 13, which further comprises:
choosing said device to be a first device, said vessel to be a first vessel, and said lid to be a first lid, and said collector stick to be a first collector stick;
choosing a second device including a second vessel containing an amount of liquid reagent, and a second lid having a second collector stick; and,
attaching said handle to said second lid after said removing.
